# EUROPEAN PATENT SPECIFICATION

(11) **EP 2 499 415 B1**
(45) Date of publication and mention of the grant of the patent: **29.06.2016**
(21) Application number: 10777091.9
(22) Date of filing: 09.11.2010
(51) Int. Cl.: F16M 13/02

(54) **MOUNTED MEDICAL DEVICE TRACK SYSTEM**
MONTIERBARES SCHIENENSYSTEM FÜR EIN MEDIZINISCHES INSTRUMENT
SYSTÈME DE RAIL POUR LE MONTAGE D'UN DISPOSITIF MÉDICAL

(30) Priority: 10.11.2009 GB 0919633
(43) Date of publication of application: 19.09.2012
(73) Proprietor: Kenex (Electro-Medical) Limited, Harlow, Essex CM20 2HS (GB)
(72) Inventor: HUNT, Charles Kenneth, Essex CM20 2HS (GB)
(74) Representative: Willett, Christopher David
(86) International application number: PCT/GB2010/051860
(87) International publication number: WO 2011/058347

(56) References cited:
- WO-A1-2006/066812
- DE-A1- 19 905 336
- DE-A1-102008 008 900
- US-A1- 2008 259 541
- Kenex (Electro-Medical) Ltd: "Monitor supsension systems -333/1 & 333/2", , 13 August 2007 (2007-08-13), XP002619150, Retrieved from the Internet: URL:http://www.kenex.co.uk/AfcStyle/Docume ntDownload.cfm?DType=DocumentItem&Document =333_Monitor_Suspensions.pdf [retrieved on 2011-01-28] -& Kenex (Electro-medical) Ltd: "Download Product brochures Monitor suspensions, Model 333", , 13 August 2007 (2007-08-13), XP002619151, Retrieved from the Internet: URL:http://www.kenex.co.uk/Downloads/index .cfm?cl=23&cls=4 [retrieved on 2011-01-28]

## Description

### Technical field

The invention relates to a mountable track, particularly ceiling mountable, comprising a carriage moveable along the track and suitable for physically supporting and providing electrical power to a medical device mounting apparatus.

### Background

Supports for medical equipment are widely employed in medical facilities, such as hospitals. Generally, the primary function is to support the weight of the equipment whilst allowing a full range of movement. In this way, medical equipment which is often quite heavy, can be moved to its desired location and orientation without medical support staff being required to support the weight of the equipment.

A common type of support takes the form of a track mountable on a surface, such as a ceiling or wall, which comprises a carriage moveable along the track. Attached to the carriage is a medical device mounting apparatus, to which may be attached a medical device or a further supporting apparatus, such as a two-part suspension arm.

Such tracks typically need to be provided with the facility for providing electrical power to any supported medical devices. Electrical power is typically provided by arranging for an electrical cable to transmit power from a fixed point energy source to the moveable carriage. As the distance over which power is to be transmitted varies as the carriage moves, some form of allowance for this must be made.

However, known methods of connecting the cable to the carriage each have significant disadvantages.

In one such method, the electrical cable starts from a fixed energy source at one end of the track and is looped several times through a number of brackets, in a helical manner, slideably mounted on a separate external track, before connecting to the carriage. This system has the major drawback that, due to the exposed nature of the cable, objects may collide with the cable causing damage.

In another known method, a biased retractable cable is employed with a device similar to a cable coiling device from a vacuum cleaner. Whilst this keeps the cable tidy it inevitably exerts an unwanted force on the carriage and introduces moving parts which can be prone to failure.

Document US 2008/259541 describes such a mountable track according to the preamble of claim 1.

### Summary of the invention

The invention relates to a mountable track comprising a carriage, moveable along the track, and for supporting and providing electrical power to a medical device mounting apparatus, and comprising an electrical power cable providing an electrical connection between an electrical power source and the carriage, wherein the cable passes from the energy source, along an inside surface of the track and loops back in the opposite direction before connecting to the carriage, wherein a partial housing for the carriage is provided such that the carriage is physically prevented from coming away from the track due to its physical size, and wherein the carriage has wheels to move freely along the track along the inside surface of the partially enclosed channels provided by the track and wherein the cable passes along the inside surface of one such channel, thus utilising the same channel as the wheels.

In this way, the cable is kept tidy, utilises no additional potentially protruding features, exerts no tension onto the carriage and involves no additional moving parts.

The track may be mountable on any appropriate supporting surface such as a wall or ceiling of a room. The track is particularly suitable for attachment to a ceiling.

The track typically comprises two parallel track members with the facility to carry the moveable carriage without it coming away from the tracks during use.

This provides a convenient and efficient use of the existing structure for cable management and can reduce the distance the track protrudes from the ceiling.

Thus, when the cable leaves the inside surface of the track to loop back it exits the channel, typically then forming a U-shape to turn back on itself before travelling in the opposite direction, e.g. now between the two track members, before connecting with the carriage.

In use, the carriage is caused to move along the track by a medical equipment user, the cable furls or unfurls accordingly from the inside of the track. No relative movement between the cable and the inside of the track is necessary, preventing any possibility of the cable becoming tangled or damaged by the carriage.

In a further preferred embodiment, the track comprises a second carriage, similar or the same as the first carriage and a second electrical power cable passing from an energy source, along another inside surface of the track before looping back in the opposite direction before connecting to the second carriage.

In this embodiment each of the two cables are conveniently passed along each of the two track members so each track member carries a cable. The two cables will pass along their respective track members in opposite directions. This allows for complete freedom of movement of the two carriages without the possibility of entanglement between the two cables.

Preferably the first and, if present, second, electrical power cables are carried within a chain-link casing, often known as an energy chain. Such a casing will typically be sized to fit snugly within the enclosed channels to further facilitate smooth furling and unfurling of the power cables.

The invention will now be illustrated, by way of example, and with reference to the following figures, in which:
Figure 1 shows an image of a prior art arrangement wherein the power cable is attached to the carriage in a looped arrangement.
Figure 2 is another image of the arrangement shown in Figure 1, with the carriage in a different position.
Figure 3 is a schematic representation of a mountable track according to the present invention showing the carriage attached to a medical device mounting apparatus.
Figure 4 is a schematic representation of the arrangement shown in Figure 3, wherein the medical device mounting apparatus has been removed.
Figure 5. is a schematic representation of the arrangement shown in Figure 3, wherein the track elements have been removed to shown the carriage and the attached power cable within an energy chain.
Figure 6 shows a schematic representation of the arrangement shown in Figure 3, from a different perspective view.
Figure 7 is a schematic representation of the arrangement shown in Figure 3 from yet another view.
Figure 8 is a schematic representation of a track according to the present invention comprising two carriages attached to respective medical device mounting apparatuses.
Figure 9 is a diagramatic representation of the arrangement shown in Figure 8 illustrating how two carriages can move independently of each other.

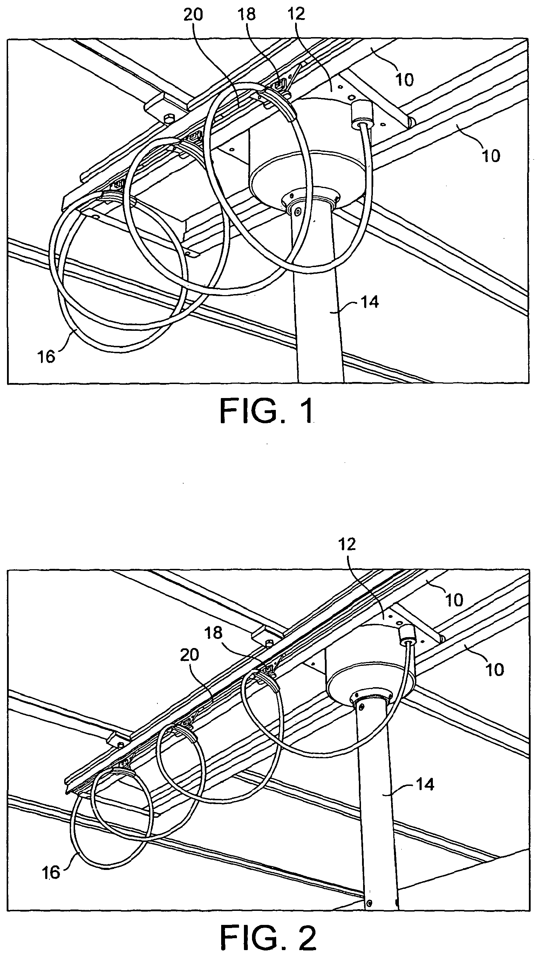

Turning to the figures, Figure 1 shows a known arrangement of mountable track mounted to a ceiling in a medical room. Track members 10 carry a carriage 12 which has wheels (not shown) which run along track members 10. Attached to the carriage is a medical device mounting apparatus 14 to which can be attached a variety of medical apparatus types, which may require electrical power. Electrical power is fed from a fixed energy source (not shown) via an electrical cable 16. The cable 16 is looped through three brackets 18, which brackets are slideably mounted on an additional external rail 20.

As can be seen from Figure 2, in use, the carriage 12 moves along tracks 10, and the looped arrangement, together with the slideably mounted brackets 18 allows the cable to expand and contract freely with the movement of the carriage. However, the exposed nature of the cable is apparent.

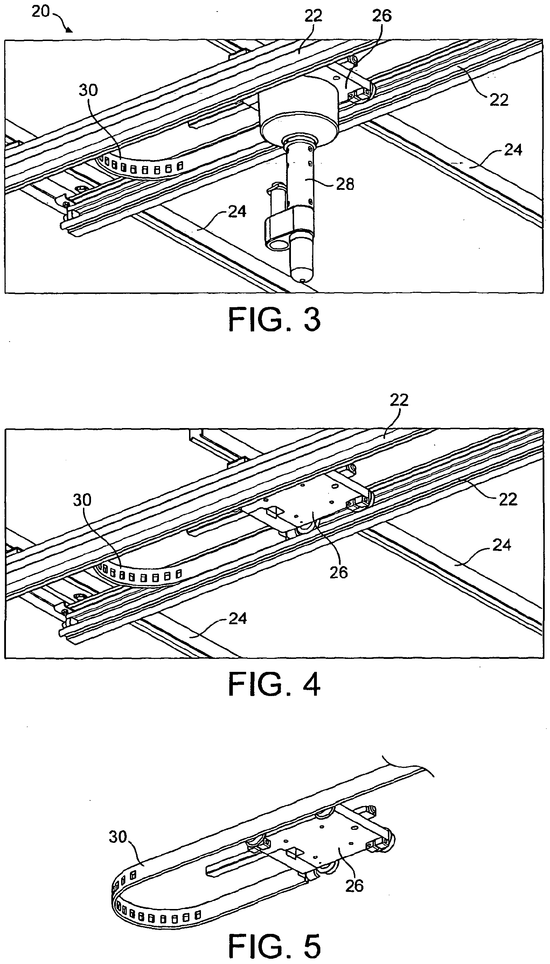

Figure 3 shows a schematic representation of a mountable track 20 according to the present invention. The track 20 comprises two track members 22 attached to mounting members 24 for attachment to a ceiling. Slideably mounted between the track members 22 is a carriage 26 comprising wheels within the partial enclosed housing provided by the tracks 22, so that it may freely move along the track. Attached to the carriage 26 is a medical device mounting apparatus 28 to which medical devices of further supporting devices may be attached. An electrical connection from the carriage through the medical device mounting apparatus is provided (not shown). Also shown is an energy chain 30 enclosing an electrical cable (not shown).

Figure 4 shows the same image as Figure 3 but wherein the medical device mounting apparatus 28 has been removed to more clearly show the carriage.

Figure 5 shows the same image but where the track members 22 have been removed to show the passage of the energy chain 30 through the track members 22. It can be seen that the energy chain runs along a track member 22 before looping back on itself and then connecting to the carriage.

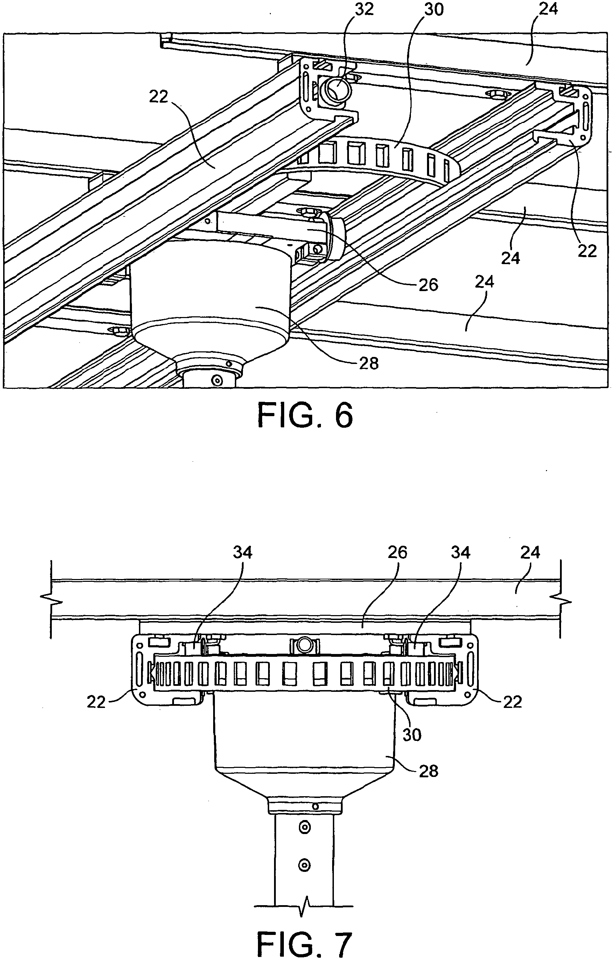

Figures 6 and 7 show a view of the end of the arrangement shown in Figure 3. As can be seen in Figure 6, there is provided an entry point 32 for another electrical cable to pass along the inside of track 22 to a second carriage (not shown). Figure 7 also shows this feature, and also more clearly shows the partial enclosed housing provided by the tracks 22 which encompasses the wheels 34 of the carriage and the power cable running along the outside of the wheels.

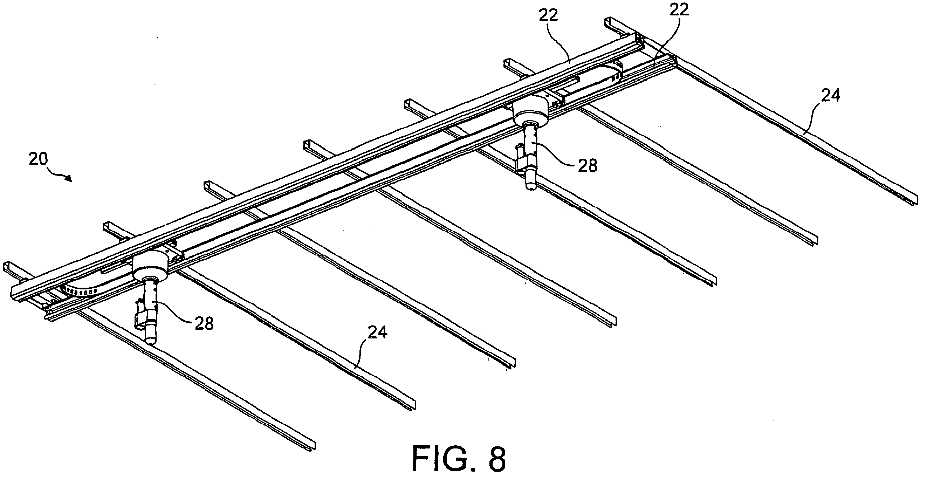

Figure 8 shows the whole of the track 20 comprising the two carriages each supporting their respective medical support mounting apparatus.

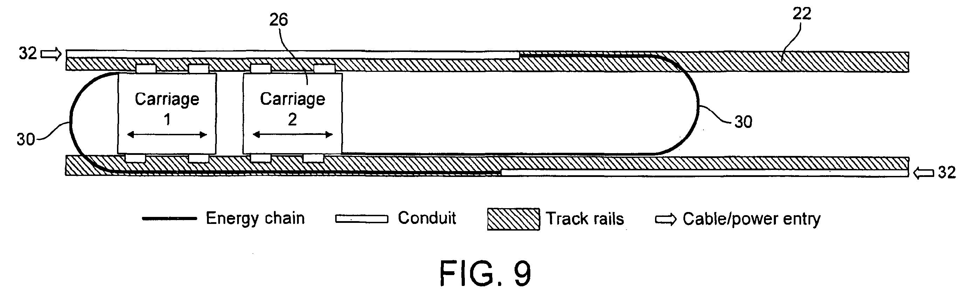

Figure 9 shows diagramatically how the electrical power enters each of the two track members 22 and passes along the inside of the track to loop back on itself before connecting with its respective carriage. As can be seen from Figure 9 movement of the carriages along the track 20 will cause the cable within its energy chain 30 to furl or unfurl accordingly and no entanglement of the cable with another cable or other surrounding equipment will occur.

## Claims

1. A mountable track (10) comprising a carriage (12), moveable along the track (10), and for supporting and providing electrical power to a medical device mounting apparatus (14), and comprising an electrical power cable (16) providing an electrical connection between an electrical power source and the carriage, wherein a partial housing for the carriage (12) provided such that the carriage (12) is physically prevented from coming away from the track (10) due to its physical size, **characterized in that** the carriage (10) has wheels (34) to move freely along the track (10) along the inside surface of partially enclosed channels provided by the track (10) and wherein the cable (16) passes from the energy source, along the inside surface of one such channel (10), thus utilising the same channel (10) as the wheels (34), and loops back before connecting to the carriage (12).

2. A track (10) according to claim 1, which is suitable for attachment to a ceiling.

3. A track (10) according to claim 1, which comprises two parallel track members (10).

4. A track according to any one of the preceding claims which comprises a second carriage (12), similar or the same as the first carriage (12) and a second electrical power cable (16) passing from an energy source, along another inside surface of the track (10) before looping back before connecting to the second carriage (12).

5. A track (10) according to any one of the preceding claims, wherein the first and, if present, second, electrical power cables (16) are carried within a chain-link casing (30).

## Patentansprüche

1. Montierbare Schiene (10), die einen entlang der Schiene (10) beweglichen Wagen (12) umfasst, und zum Halten einer Montageeinrichtung (14) für eine medizinische Vorrichtung und zum Zuführen von elektrischem Strom zu dieser, und umfassend ein elektrisches Stromkabel (16) zum Zuführen einer elektrischen Verbindung zwischen einer elektrischen Stromquelle und dem Wagen, wobei ein Teilgehäuse für den Wagen (12) derart vorgesehen ist, dass der Wagen (12), aufgrund seiner physischen Größe, physisch daran gehindert wird, die Schiene (10) zu verlassen, **dadurch gekennzeichnet, dass** der Wagen (10) Räder (34) aufweist, um sich frei entlang der Schiene (10) entlang der inneren Oberfläche von teilweise umschlossenen Kanälen zu bewegen, die von der Schiene (10) bereitgestellt werden, und wobei das Kabel (16) von der Energiequelle entlang der inneren Oberfläche eines derartigen Kanals (10) verläuft, womit derselbe Kanal (10) wie von den Rädern (34) verwendet wird, und vor dem Verbinden mit dem Wagen (12) zurückschleift.

2. Schiene (10) nach Anspruch 1, die zum Anbringen an einer Raumdecke geeignet ist.

3. Schiene (10) nach Anspruch 1, die zwei parallele Schienenglieder (10) umfasst.

4. Schiene nach einem der vorhergehenden Ansprüche, umfassend einen zweiten Wagen (12), der dem ersten Wagen (12) ähnlich oder gleich ist, und ein zweites elektrisches Stromkabel (16), das von einer Energiequelle entlang einer anderen inneren Oberfläche der Schiene (10) verläuft, bevor es vor dem Verbinden mit dem zweiten Wagen (12) zurückschleift.

5. Schiene (10) nach einem der vorhergehenden Ansprüche, wobei das erste und, falls vorhanden, das zweite elektrische Stromkabel (16) in einem Kabelschlepp (30) getragen werden.

## Revendications

1. Rail (10) pouvant être monté, comprenant un chariot (12) déplaçable le long du rail (10) et servant à supporter et fournir de la puissance électrique à un appareil de montage de dispositif médical (14), comprenant un câble d'alimentation électrique (16) assurant une connexion électrique entre la source de puissance électrique et le chariot, un boîtier partiel pour le chariot (12) étant prévu de manière à empêcher physiquement le chariot (12) de sortir du rail (10) du fait de sa dimension physique, **caractérisé en ce que** le chariot (10) présente des roues (34) destinées à se déplacer librement le long du rail (10) le long de la surface intérieure de canaux partiellement fermés constitués par le rail (10) et le câble (16) passant depuis la source d'énergie, le long de la surface intérieure d'un tel canal (10), en utilisant ainsi le même canal (10) que les roues (34), et revenant en boucle avant de se raccorder au chariot (12).

2. Rail (10) selon la revendication 1, qui est adapté pour être fixé à un plafond.

3. Rail (10) selon la revendication 1, comprenant deux organes de rail parallèle (10).

4. Rail selon l'une quelconque des revendications précédentes, comprenant un deuxième chariot (12) similaire ou identique au premier chariot (12) et un deuxième câble d'alimentation électrique (16) passant depuis une source d'énergie, le long d'une autre surface intérieure du rail (10) avant de revenir en boucle avant de se raccorder au deuxième chariot (12).

5. Rail (10) selon l'une quelconque des revendications précédentes, dans lequel le premier, et le cas échéant, le deuxième câble d'alimentation électrique (16) sont portés à l'intérieur d'un boîtier à mailles de chaîne (30).
